# EUROPÄISCHE PATENTSCHRIFT

(11) **EP 2 732 830 B1**
(45) Veröffentlichungstag und Bekanntmachung des Hinweises auf die Patenterteilung: **02.09.2020**
(21) Anmeldenummer: 13189558.3
(22) Anmeldetag: 21.10.2013
(51) Int. Cl.: A61L 24/06, A61L 24/02, A61L 24/00

(54) **Antiseptischer Polymethylmethacrylat-Knochenzement**
Antiseptic polymethyl methacrylate bone cement
Ciment osseux en polyméthylméthacrylate antiseptique

(30) Priorität: 16.11.2012 DE 102012022419
(43) Veröffentlichungstag der Anmeldung: 21.05.2014
(73) Patentinhaber: Heraeus Medical GmbH, 61273 Wehrheim (DE)
(72) Erfinder: Vogt, Sebastian, 99092 Erfurt (DE); Kühn, Klaus-Dieter, 35041 Marburg-Elnhausen (DE)
(74) Vertreter: Heraeus IP

(56) Entgegenhaltungen:
- EP-A2- 0 190 504
- EP-A2- 2 198 893
- WO-A1-2005/009495
- DE-A1-102005 023 094
- PENNER M J ET AL: "Elution characteristics of vancomycin and tobramycin combined in acrylic bone-cement", THE JOURNAL OF ARTHROPLASTY, CHURCHILL LIVINGSTONE, AMSTERDAM, NL, Bd. 11, Nr. 8, 1. Dezember 1996 (1996-12-01), Seiten 939-944, XP004762859, ISSN: 0883-5403, DOI: 10.1016/S0883-5403(96)80135-5

## Beschreibung

Es wird eine antiseptische Zusammensetzung zur Verwendung als Knochenzement, insbesondere ein antiseptischer Polymethylmethacrylat-Knochenzement vorgeschlagen, der härtbar ist und einen Gehalt mindestens einer Komponente aufweist, die eine oxidierend wirkende Verbindung ist oder aus der eine oxidierend wirkende Verbindung freisetzbar ist. Vorzugsweise wird Wasserstoffperoxid freigesetzt oder ist freisetzbar. Dabei wird besonders vorzugsweise ein Addukt oder Salz des Wasserstoffperoxids eingesetzt, das in Gegenwart von Wasser oder im wässrigen Milieu Wasserstoffperoxid freisetzt. Der antiseptische Polymethyl-methacrylat-Knochenzement kann zur mechanischen Fixierung von primären Totalgelenkendoprothesen, zur mechanischen Fixierung von Revisions-Total-gelenkendoprothesen und zur Herstellung von Spacern verwendet werden.

Gegenstand der Erfindung ist ein antiseptischer Polymethylmethacrylat-Knochenzement. Dieser Zement ist besonders für die Herstellung von Spacern (temporären Platzhaltern) im Rahmen der zweizeitigen septischen Revision von Gelenk-Totalendoprothesen (TEP) bestimmt.

Gelenk-Totalendoprothesen werden in großem Umfang zum Erhalt der Gelenkfunktionen vor allem beim Knie- und dem Hüftgelenk eingesetzt. Daneben werden in geringerem Umfang auch Schulter-TEP und Ellenbogen-TEP verwendet. Die TEP können mit Polymethylmethacrlyat-Knochenzement oder auch press-fit im Fall der zementfreien Prothesen im knöchernen Implantatlager verankert werden.

Es kommt leider nach der Implantation von TEP, trotz moderner Hygiene und OP-Techniken, in sehr geringem Umfang zu Infektionen am Knochen- bzw. Weichgewebe, welches die TEP umgibt. Dabei werden Früh- und Spätinfektionen unterschieden. Die Frühinfektionen werden vielfach durch Keime verursacht, die während der TEP-Implantation in das humane Gewebe gelangt sind. Bei den Spätinfektionen wird im Allgemeinen eine hämatogene Streuung von Keimen diskutiert. Diese Infektionen stellen für den Patienten eine sehr schwerwiegende Komplikation dar. TEP-assoziierte Infektionen können im Extremfall zu einer chronischen Osteitis und sogar in extremsten Fällen zur lebensbedrohenden Sepsis führen.

TEP-assoziierte Infektionen werden hauptsächlich durch einzeitigen und zweizeitigen TEP-Wechsel behandelt. Wesentlich ist dabei in beiden Fällen, dass das infizierte Gewebe radikal im Rahmen eines Debridements abgetragen wird. Im Fall des einzeitigen TEP-Wechsels wird unmittelbar nach Explantation der primären TEP und nachfolgendem radikalen Debridement die Revisions-TEP mit Hilfe eines mit Antibiotika dotierten Polymethylmethacrylat-Knochenzements im debridierten knöchernen Implantatlager mechanisch fixiert. Im Fall des zweizeitigen TEP-Wechsels wird in einer ersten OP die infizierte primäre TEP entfernt, das infizierte Gewebe debridiert und anschließend wird ein aus Polymethylmethacrylat bestehender temporärer Platzhalter (Spacer), der ein Antibiotikum oder, wie in den meisten Fällen, zwei oder mehrere Antibiotika enthält, implantiert. Dieser Spacer bildet die entfernte primäre TEP nach und dient zum sogenannten Dead-Space-Management. Das bedeutet, der Spacer verhindert die Ausbildung von ausgedehnten Hämatomen, die ein Rezidiv begünstigen können, und verhindert weitgehend eine Atrophie der Muskulatur und des Bandapparates. Weiterhin gibt der antibiotisch dotierte Spacer kontinuierlich die enthaltenden Antibiotika an das umliegende Gewebe ab. Dadurch werden eventuell noch vorhandene restliche mikrobielle Keime in dem umliegenden Gewebe und vor allem an der Spaceroberfläche wirksam bekämpft. Nach ca. 4-6 Wochen nach der Implantation des Spacers wird üblicherweise das Gelenk punktiert und anschließend wird das Punktat auf das Vorhandensein von mikrobiellen Keimen geprüft. Daneben werden die für Entzündungen typischen klinischen Marker wie CRP, Rötung, Schwellung und Erwärmung erfasst. Wenn eine Beruhigung der Infektion festgestellt wurde, dann erfolgt in einer zweiten OP die Entfernung des Spacers und es wird nochmals debridiert. Anschließend wird die Revisions-TEP zementfrei oder auch unter Verwendung von Polymethylmethacrylat-Knochenzement implantiert.

Bei der Herstellung von Spacern werden normalerweise Kombinationen von Antibiotika dem Zementpulver zugesetzt, die entsprechend den für die Infektion ursächlichen mikrobiellen Keimen ausgewählt werden (L. Frommelt: Lokale Antibiotikatherapie. In: Septische Knochenchirurgie. Hrsg. R. Schnettler, H.-U. Steinau, Georg Thieme Verlag Stuttgart New York 2004, 82-90). Dabei sind Kombinationen von zwei bis vier unterschiedlichen Antibiotika üblich. Es gibt jedoch immer wieder kritische Diskussionen zur Verwendung von Antibiotika im Hinblick auf Resistenzbildungen. Vorteilhaft ist jedoch, dass viele der relevanten Antibiotika, zum Beispiel Gentamicin und Vancomycin, praktisch unverändert vom humanen Organismus renal ausgeschieden werden können

Es gab Bemühungen Antiseptika anstelle von Antibiotika in Polymethylmethacrylat-Knochenzemente zu integrieren.

So wurde im WO 8201990 A1 ein Knochenzement vorgeschlagen, der bis zu 5 Gew.-% Silbersalze enthält. Eine antimikrobielle Zusammensetzung, die bis zu 10 Gew.-% elementares Silber und zusätzlich noch Titandioxid oder Tantaloxid enthält, wurde im US 4849223 A offengelegt. Im EP 1313518 A1 wurde ein Knochenzement beschrieben, der Silberpartikel mit einer Größe von 20 µm enthält. Diese Silberpartikel sind aus kleineren Silberpartikeln, mit einer Größe im Nanometerbereich, aufgebaut.

Kritisch ist bei der Verwendung von elementaren Silber oder auch Silbersalzen, dass die eigentlich mikrobiozid wirkenden Silber-Ionen unselektiv neben mikrobiellen Strukturen auch mit Strukturen des humanen Gewebes wechselwirken. So ist die Bildung von in Wasser gering löslichen Salzen mit Cystein und Cystein enthaltenden Proteinen möglich. Weiterhin können Silber-Ionen mit Phosphat-Ionen unter Bildung des schwer löslichen Silberorthophosphats reagieren. Es ist davon auszugehen, dass in den humanen Körper eingebrachte Silberverbindungen praktisch nicht ausgeschieden werden können, wie die bereits seit langem bekannte Erscheinung der Argyrie, der irreversiblen Hautverfärbung durch Silber, zeigt.

Andere antiseptische Strategien sind in WO2005/009495 und EP2198893 beschrieben. Im EP 1648531 A1 wurde ein Polymethylmethacrylat-Knochenzement offenbart, der kationische Antiseptika enthält, wobei besonders das Antiseptikum Polyhexamethylenbiguanid bevorzugt wird. Kritsch muss hierbei jedoch diskutiert werden, dass diese kationischen Antiseptika vom humanen Gewebe nicht abgebaut werden können und die Gefahr einer lokalen Akkumulation besteht.

Der Erfindung liegt die Aufgabe zu Grunde einen Knochenzement, vorzugsweise einen Polymethylmethacrylat-Knochenzement zu entwickeln, der nach einer Implantation durch Einwirkung von Körperflüssigkeiten ein breit wirksames Antiseptikum freisetzt, wobei das Antiseptikum einerseits temporär eine hohe mikrobiozide Wirkung haben soll und sich andererseits das Antiseptikum nicht im humanen Gewebe akkumulieren soll bzw. darf, um unerwünschte toxische Effekte zu vermeiden. Das Antiseptikum soll weiterhin möglichst retardiert freigesetzt werden.

Die Aufgabe der Erfindung wurde gemäß der Ansprüche gelöst. Bevorzugte Ausführungsformen sind in den Unteransprüchen und in der Beschreibung detailliert beschrieben.

Die Erfindung basiert auf der Idee mindestens eine Wasserstoffperoxid-Verbindung in eine Zusammensetzung zur Verwendung als Knochenzement, wie einen Polymethacrylat-Knochenzement einzubringen, die in der Lage ist unter definierten Bedingungen Wasserstoffperoxid als oxidierend wirkende Verbindung vorzugsweise retardiert freizusetzen. Dabei werden besonders bevorzugt ein Addukt und/oder ein Salz des Wasserstoffperoxids in den Polymethylmethacrylat-Knochenzement eingebracht. Bevorzugte Addukte und Salze setzen durch Einwirkung von Wasser oder wässrigen Lösungen, wie einem wässrigen Milieu im Körperinneren, Wasserstoffperoxid frei. Wasserstoffperoxid ist ein breit wirksames Antiseptikum, das infolge seiner oxidierenden Wirkung eine stark mikrobiozide Wirkung hat. Besonders vorteilhaft ist, dass sich Wasserstoffperoxid nicht im humanen Organismus akkumulieren kann, weil im humanen Gewebe Katalasen und Peroxidasen vorhanden sind, die Wasserstoffperoxid zersetzen. Der besondere Vorteil der erfindungsgemäßen Zusammensetzung oder des erfindungsgemäßen Knochenzementes ist die Vermeidung der Resistenzbildung in den Mikroorganismen, wie es bei Antibiotika basierten Therapien möglich ist. Ein weiterer Vorteil der erfindungsgemäßen Verwendung ist die sehr lokale antiseptische Wirkung, da die vorgenannten Katalsen und Peroxidasen das gebildete Wasserstoffperoxid wieder abbauen könne.

Gegenstand der Erfindung ist eine Zusammensetzung zur Verwendung als Knochenzement, insbesondere ein Polymethacrylat-Knochenzement, wobei die Zusammensetzung härtbar ist und einen Gehalt mindestens einer Komponente aufweist, die eine oxidierend wirkende Verbindung ist oder aus der eine oxidierend wirkende Verbindung freisetzbar ist, wobei die oxidierende Verbindung in Gegenwart von Wasser aus der Komponente freigesetzt wird. Gleichfalls Gegenstand der Erfindung ist die Verwendung mindestens einer oxidierend wirkenden Verbindung in einem antiseptischen Knochenzement. Vorzugsweise wird aus der Komponente die oxidierend wirkende Verbindung in Gegenwart von H₂O, wie Feuchte, Wasser oder im wässrigen Milieu einer Körperflüssigkeit, als Wasserstoffperoxid freigesetzt. Daher gelten als oxidierend wirkende Verbindungen gemäß der Erfindung Wasserstoffperoxid oder mindestens eine Peroxy-Gruppe (-O-O-) enthaltende Verbindungen, die mit Wasser unter Freisetzung von Wasserstoffperoxid (H₂O₂, H-O-O-H) reagieren.

Erfindungsgemäß ist die Zusammensetzung ein antiseptischer Polymethylmethacrylat-Knochenzement.

Das retardiert freigesetzte Wasserstoffperoxid kann als breit wirksames Antiseptikum oberflächliche Keime auf Spacern oder Endoprothesen reduzieren, und selbst im Körper durch Katalase (Gen-Name: CAT), einem Enzym, das Wasserstoffperoxid (H₂O₂) zu Sauerstoff (O₂) und Wasser (H₂O) umsetzt, abgebaut werden. Auf diese Weise ist die antimikrobielle Wirksamkeit lokal im Bereich des Implantats, Spacers oder der Endoprothese beschränkt und akkumuliert sich nicht im Körper des Patienten.

Ebenso Gegenstand der Erfindung ist eine Zusammensetzung zur Verwendung als Knochenzement, insbesondere ein Polymethacrylat-Knochenzement, wobei die Zusammensetzung härtbar ist und Wasserstoffperoxid freisetzt oder wobei aus der Zusammensetzung Wasserstoffperoxid freisetzbar ist bzw. abgegeben werden kann. Besonders bevorzugt wird Wasserstoffperoxid retardiert aus der härtbaren oder der gehärteten Zusammensetzung freigesetzt. Vorzugsweise innerhalb von bis zu 4 oder auch bis zu 6 Wochen, insbesondere wird ein Teil des Wasserstoffperoxids unmittelbar freigesetzt, um vorhandene Keime sofort abzutöten sowie vorzugsweise in ggf. geringerer Konzentration über den anschließenden Zeitraum, wobei die Konzentration weiter abnehmen kann.

Ebenso Gegenstand der Erfindung ist eine Zusammensetzung zur Verwendung als Knochenzement, insbesondere ein Polymethacrylat-Knochenzement, besonders bevorzugt ein antiseptischer Knochenzement. Dabei ist die Zusammensetzung härtbar und enthält, vorzugsweise als Komponente eine Wasserstoffperoxid-Verbindung, die Wasserstoffperoxid ist oder Wasserstoffperoxid freisetzt. Bevorzugt ist die Wasserstoffperoxid-Verbindung ausgewählt aus Wasserstoffperoxid, einem Wasserstoffperoxid Addukt, einem Wasserstoffperoxid Komplex, einem Salz von Wasserstoffperoxid, einem Co-Kristall mit Wasserstoffperoxid, einer Wasserstoffperoxid freisetzenden Verbindung, wie bspw. einer enzymatisch Wasserstoffperoxid freisetzenden Verbindung, einer festen Wasserstoffperoxid enthaltenden Lösung, die eine nicht-stöchiometrische Zusammensetzung aufweist, oder einem Gemisch enthaltend mindestens zwei der vorgenannten Verbindungen. Erfindungsgemäß ist das Wasserstoffperoxid aus der Wasserstoffperoxid-Verbindung freisetzbar, vorzugsweise nach einer Härtung, wie durch Polymerisation eines flüssigen Monomers und mindestens einem in dem Monomer löslichen organischen Polymer.

Die vorgenannten Wasserstoffperoxid-Verbindungen, vorzugsweise die vorgenannten Addukte, Salze, Komplex und/oder Co-Kristalle, können generell jeweils unabhängig als Polymorphe, Hydrate, wie stöchiometrische oder nicht-stöchiometrische Hydrate als auch als Solvate oder amorph in der Zusammensetzung eingesetzt werden. Bei der Verwendung von Salzen von Wasserstoffperoxid oder Wasserstoffperoxid-Addukten, -Komplexen oder Co-Kristallen sind generell alle pharmazeutisch verträglichen oder arzneimittelrechtlich zugelassenen Verbindungen als zweite Molekülverbindung oder als organische oder anorganische ionische Verbindung in den vorgenannten Wasserstoffperoxid-Verbindungen geeignet. Bevorzugt sind die Kationen von Natrium, Kalium, Magnesium, Calcium und/oder Zink.

Generell werden in der Zusammensetzung, vorzugsweise dem Knochenzement, als Wasserstoffperoxid-Verbindungen pharmakologisch verträgliche Verbindungen eingesetzt. Beispiele dafür sind Glukonate, Maleate etc.

Unter dem Begriff Polymethylmethacrylat-Knochenzement werden konventionelle Zemente verstanden, bei denen durch Vermischung einer Polymerpulver-Komponente und einer flüssigen Monomerkomponente ein durch radikalische Polymerisation selbst aushärtender Zementteig gebildet wird. Daneben fallen unter den Begriff auch pastenförmige Polymethylmethacrylat-Knochenzemente, bei denen durch Vermischung von zwei separaten vorgequollenen Zementpasten ein selbsthärtender Zementteig gebildet wird. Exemplarisch sind dafür die Offenlegungsschriften DE 102007050762 B3, DE 102010024653 B4 und DE 102010005956 B4.

Wir beschreiben als Knochenzement verwendbare Zusammensetzungen mit bevorzugt mindestens a) Wasserstoffperoxid, ein Wasserstoffperoxid-Addukt, einen Wasserstoffperoxid-Komplex oder ein Wasserstoffperoxid-Salz, das in Gegenwart von Feuchte, Wasser, wässrigem Mileu oder einer wässrigen Lösung Wasserstoffperoxid als oxidierend wirkende Verbindung freisetzt und/oder b) eine Wasserstoffperoxid-Verbindung, die durch Enzyme katalysiert Wasserstoffperoxid freisetzt. Daher kann nach einer Alternative auch als eine Wasserstoffperoxid freisetzende Verbindung auch eine Wasserstoffperoxid-Verbindung eingesetzt werden, die durch Anwesenheit eines Enzyms Wasserstoffperoxid freisetzt.

Besonders bevorzugt ist ein antiseptischer Polymethylmethacrylat-Knochenzement umfassend mindestens ein Addukt oder Salz des Wasserstoffperoxids, das in Gegenwart von Wasser, Feuchte, in wässrigen Lösungen oder einem wässrigen Milieu Wasserstoffperoxid freisetzt, insbesondere aus dem gehärteten Zustand.

Weiter ist es bevorzugt, wenn als Wasserstoffperoxid-Verbindung keine sauer reagierende Verbindung oder keine Peroxysäure, wie Peroxyessigsäure eingesetzt wird. Generell kann aber ein Salz einer Peroxysäure eingesetzt werden, wie ein Alkali- oder Erdalkalimetall-Salz oder ZinkSalz von Peroxysäuren, die auch als Puffer wirken.

Wir beschreiben Komponenten, die mindestens eine Wasserstoffperoxid-Verbindung umfasst mindestens ein Harnstoff-Wasserstoffperoxid-Addukt (CAS 124-43-6) und/oder ein Natriumpercarbonat (CAS 15630-89-4, ein Addukt von Wasserstoffperoxid mit Natriumcarbonat.

Die erfindungsgemässen Wasserstoffperoxidverbindungen sind die Wasserstoffperoxid-Salze Calciumperoxid (CAS 1305-79-9), und Calciumperoxid-Octahydrat (CAS 78403-22-2). Das Calciumperoxid setzt dagegen in Gegenwart von Wasser langsamer Wasserstoffperoxid frei. Bei dieser Hydrolyse wird neben Wasserstoffperoxid auch Calciumhydroxid gebildet. Das Calciumhydroxid ist eine mittelstarke Base, die mit Pufferubstanzen, insbesondere den Alkalisalzen der Puffersubstanzen abgefangen werden kann, bspw. unter Bildung unlöslicher Salze.

Durch eine Kombination von Wasserstoffperoxid-Verbindungen mit unterschiedlichen aber definierten Freisetzungsprofilen von Wasserstoffperoxid, kann die Freisetzungsgeschwindigkeit, die Dauer der Freisetzung und die lokale Konzentration an Wasserstoffperoxid an der Oberfläche bzw. in dem die ausgehärteten Zusammensetzung umgebenden Gewebe, wie einen Implantat oder Spacer, sehr gut je nach Bedarf gesteuert werden.

Zur Einstellung der gewünschten Freisetzung von Wasserstoffperoxid, kann in der Zusammensetzung, insbesondere in dem Knochenzement, die Wasserstoffperoxid-Verbindung zu 0,001 bis 10 Gew.-%, insbesondere 0,01 bis 5 Gew.-%, bevorzugt von 0,01 bis 2,5 Gew.-%, weiter bevorzugt zwischen 0,01 bis 1 Gew.-% in der Gesamtzusammensetzung enthalten sein. Dabei kann die Zusammensetzung auch durch Mischen von zwei oder mehr Pasten oder einer Paste und einer Pulverkomponente hergestellt werden, wobei die Wasserstoffperoxid-Verbindung in einer oder beiden Pasten sowie auch in der Pulverzusammensetzung enthalten sein kann. Dabei ist es bevorzugt, wenn die Wasserstoffperoxid-Verbindung mit einem Gehalt in der Zusammensetzung enthalten ist, der nach einer Aushärtung einem Gehalt von etwa 0,01 bis 5 Gew.-% entspricht. Erfindungsgemäß ist mindestens ein Addukt oder Salz des Wasserstoffperoxids im ausgehärteten Polymethylmethacrylat-Knochenzement in einem Bereich von 0,01 bis 5 Gew.-%, vorzugsweise zwischen 0,01 bis 1 Gew.-% enthalten.

Unter einem Addukt (http://goldbook.iupac.org/A00138.html)-Addukt wird eine chemische Spezies AB verstanden, die Moleküle durch direkte Kombination von zwei getrennten Molekülen A und B in der Weise umfasst, die durch eine Veränderung möglicher Koordinationen gebildet wird, aber keinen Verlust, von Atomen in den Molekülen A und B bewirkt. Ein Addukt kann andere Eigenschaften besitzen als die einzelnen Moleküle. Die Stöchiometrie von Addukten kann 1:1 aber auch jede andere denkbare Stöchiometrie sein, wie bspw. ein Bis-Addukt (2:1) oder 2:3 Addukt etc. Ein intramolekulares Addukt kann gebildet werden, wenn die Gruppen A und B innerhalb der gleichen molekularen Entität enthalten sind. Bekannt sind auch Lewis-Addukte, π-Addukte. σ-Addukt oder Komplexe (http://goldbook.iupac.org/ C01203.html). Unter einem Komplex wird eine molekulare Einheit verstanden, die sich durch lose Assoziation von zwei oder mehr molekularen Verbindungen bildt, die ionisch oder ungeladen sein können. Bekannt ist u.a. auch der Begriff Elektronen-Donor-Akzeptor-Komplex. Als Co-Kristall wird eine kristalline Verbindung verstanden, in der eine feste Stöchiometrie der beteiligten Moleküle vorliegt und, die in der Regel über Wasserstoffbrückenbindungen oder schwache Wechselwirkungen wie bspw. (π)C-H-O/N-, -(O)C-H-O/N-Bindungen ausbildet sind.

Als Wasserstoffperoxid-Addukte können beispielsweise auch pharmazeutisch verträgliche Addukte mit Natriumacetat, Natriumcitrat, Succinimid, Asparagin, Hexamethylentetratramin sowie weitere Addukte von Wasserstoffperoxid mit pharmazeutisch verträglichen Molekülen oder Salzen eingesetzt werden.

Es wurden zahlreiche Vorschläge gemacht, die Lagerstabilität von Wasserstoffperoxid-Addukten zu erhöhen; In DE 681205 C wurde vorgeschlagen, das Wasserstoffperoxid-Harnstoff-Addukt mit wasserabstoßenden Mitteln z.B. mit Fetten oder Wachsen zu imprägnieren. In DE 687217 C wurde der Zusatz von oberflächenaktiven Stoffen beschrieben. Nach U 3629331 A wird der Zusatz von Ethylendiamintetraessigsäure (EDTA) und Natriumdihydrogenphosphat zum Harnstoff-Peroxid empfohlen. Zur Verbesserung des Fließverhaltens können außerdem 3 bis 9 Gewichtsprozent kolloidaler Kieselsäure zugesetzt werden. In DE 1169901 B wird ein Verfahren zur Herstellung von Wasserstoffperoxid enthaltenden Pasten beschrieben, bei welchem Wasserstoffperoxid oder Harnstoffperhydrat in Polyethylenoxiden gelöst werden.

Weiter bevorzugt ist es, wenn die Zusammensetzung mindestens eine Puffersubstanz umfasst, vorzugsweise eine Puffersubstanz aus der Gruppe der primären Alkaliphosphate, der sekundären Alkaliphosphate, der primären Alkalicitrate, der sekundären Alkalicitrate und/oder einem Salz von Carbonsäuren, insbesondere mit 1 bis 20 C-Atomen, vorzugsweise ein Salz mindestens einer Fruchtsäure, ein Salz einer alpha-Hydroxysäure und/oder ein Salz einer Fettsäure, insbesondere mit 1 bis 20 C-Atomen. Besonders bevorzugte Salze der vorgenannten Säuren sind die Alkali-, Erdalkali- und/oder Zink-Salze der Citronensäure, Aepfelsäure, Weinsäure und/oder Milchsäure. Besonders bevorzugte Salze umfassen die Kationen von Natrium, Kalium, Magnesium und/oder Zink der vorgenannten Phosphate oder Carboxylate.

Somit kann ein Salz, insbesondere ein wasserlösliches Salz, einer Fruchtsäure, bspw. aus der Gruppe Citronensäure, Aepfelsäure, Salicylsäure, sowie auch der der Weinsäure und der Milchsäure im antiseptischen Polymethylmethacrylat-Knochenzement enthalten sein. Durch diese Puffersubstanz kann bei Verwendung geeigneter Mengen das bei der Hydrolyse von Calciumperoxid gebildete Calciumhydroxid neutralisiert werden. Ebenso geeignet können Alkali-, Erdalkalisalze, Zink-Salze von α-Hydroxycarbonsäuren oder Fettsäuren mit 1 bis 8 C-Atomen als Puffersubstanzen eingesetzt werden.

Dabei kann die Puffersubstanz zur Steuerung des pH-Wertes und/oder zur Steuerung der Freisetzung des Wasserstoffperoxids zusammen mit der Wasserstoffperoxid-Verbindung vorliegen als i) ein Addukt mit einer Puffersubstanz, ii) in einem Co-Kristall zusammen mit der Puffersubstanz, iii) in einer Formulierung zusammen mit mindestens einer Puffersubstanz, insbesondere als Granulat, Kompaktat, Extrudat, in einer Matrix, einer beschichteten Formulierung, einem umhüllten Pellet, oder iv) im Gemisch mit mindestens einer Puffersubstanz, jeweils optional in Gegenwart einer weiteren Puffersubstanz und/oder weiteren üblichen pharmazeutischen Hilfsstoffen und/oder retardierend wirkenden Beschichtungsmitteln.

Gemäß einer weiteren Ausführungsform kann die Wasserstoffperoxid-Verbindung in einem durch H₂O quellbarem, insbesondere porenbildenden oder erodierenden Acrylat eingebettet oder davon umhüllt ein, um die ohnehin retardierte Freisetzungskinetik noch stärker zu steuern. Typische Retard-Acrylate sind bspw. EUDRAGIT® Pulvermassen.

Besonders vorteilhaft ist es, wenn das Calciumperoxid mit der Puffersubstanz zusammen granuliert wurde und diese Granulate dem Polymethylmethacrylat-Knochenzement zugesetzt werden. Der besondere Vorteil liegt darin, dass in jedem Granulum eine Puffersubstanz enthalten ist, die während der Hydrolyse des Calciumperoxids den pH-Wert stabilisiert. Dadurch werden lokale basische Reaktionen weitgehend vermieden. Der Gehalt der Puffersubstanz in der Zusammensetzung kann zwischen 0,001 bis 10 Gew.-%, bevorzugt von 0,001 bis 5 Gew.-%, besonders bevorzugt von 0,001 bis 2,5 Gew.-%, weiter bevorzugt zwischen 0,001 bis 1 Gew.-% in Bezug auf die Gesamtzusammensetzung betragen. In einer Formulierung zusammen mit der Wasserstoffperoxid-Verbindung kann die Konzentration geringer sein als bei einem einfachen Vermischen der Wasserstoffperoxid-Verbindung und der Puffersubstanz mit dem Polymer und Monomer.

Die erfindungsgemäßen Zusammensetzungen sind Knochenzemente umfassend mindestens ein organisches Polymer oder Mischungen von organischen Polymeren, die insbesondere in den Monomeren löslich sind, wobei die Polymere Polyacrylate sind. Insbesondere ist das organische Polymer ausgewählt aus Poly(alkyl-2-acrylsäurealkylester), Poly(aryl-2-acrylsäurealkylester), Poly(arylalkyl-2-acrylsäurealkylester) jeweils unabhängig mit 1 bis 20 C-Atomen, vorzugsweise 1 bis 18 C-Atomen in der Alkyl-Gruppe, insbesondere mit 1 bis 4 C-Atomen, jeweils unabhängig mit 6 bis 13 C-Atomen in der Aryl-Gruppe, insbesondere mit 6, 10, 12 oder 13 C-Atomen, jeweils unabhängig mit 6 bis 14 C-Atomen in der Arylalkyl-Gruppe, insbesondere mit 8 bis 12 C-Atomen, und jeweils unabhängig mit 1 bis 10 C-Atomen in der Alkylester-Gruppen, insbesondere mit 1 bis 4 C-Atome, oder eine Mischung umfassend mindestens zwei der genannten Polymere.

Besonders bevorzugt ist das organische Polymer, insbesondere ein in dem Monomer lösliches Polymer, ausgewählt aus der Gruppe Poly(methacrylsäuremethylester), Poly(methacrylsäureethylester), Poly(methylmeth-acrylsäurepropylester), Poly(methacrylsäureisopropylester), Poly(methylmeth-acrylat-co-methyl-acrylat), Poly(styrol-co-methylmeth-acrylat), Copolymeren der genannten Verbindungen und einer Mischung umfassend mindestens zwei dieser Polymere, wobei Polymethmethacrylat (PMMA) besonders bevorzugt eingesetzt wird,

Als in dem mindestens einen radikalisch polymerisierbaren Monomer lösliches Polymer wird ein Polymer verstanden, das sich in diesem polymerisierbaren Monomer zu wenigstens 10 g/l, vorzugsweise zu wenigstens 25 g/l, besonders bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse (Mw) von wenigstens 150.000 g/mol, insbesondere mindestens 200.000 g/mol bis größer gleich 5000.000 g/mol. Beispielsweise kann es sich bei dem löslichen Polymer um ein Polymer oder Copolymer eines Methacrylsäureesters handeln. Gemäß einer besonders bevorzugten Ausführungsform ist das wenigstens eine lösliche Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Die Menge des in dem radikalisch polymerisierbaren Monomer löslichen Polymers in der erfindungsgemäßen Zusammensetzung liegt üblicherweise in einem Bereich von 1 bis 85 Gew.-%, bezogen auf das Gesamtgewicht der erfindungsgemäßen Zusammensetzung. Entsprechend kann der Gehalt an Polymer jeweils unabhängig in einer der nachstehenden Pasten A und/oder B sowie der Pulverkomponente C und/oder der Monomerkomponente D von 1 bis 85 Gew.-% in Bezug auf die jeweilige Gesamtzusammensetzung an Paste, Pulverkomponente oder Monomerkomponente betragen.

Als besonders bevorzugtes organisches Polymer wird mindestens ein Poly(methacrylsäuremethylester), (PMMA) und als Monomer Methacrylsäuremethylester (MMA) eingesetzt, wobei auch Gemische dieser mit weiteren Monomeren oder ein Co-Polymer von PMMA eingesetzt werden können.

Als in den Monomeren lösliche Polymere, insbesondere Polyacrylate, werden Polymere mit einem Molekulargewicht (Mw) von vorzugsweise größer gleich 200.000 g/mol zur Herstellung von Pulverkomponenten eingesetzt, bevorzugt sind Molekulargewichte von größer gleich 500.000 g/mol. Zur Verwendung in Pasten können auch Polymere mit einem Molekulargewicht von kleiner gleich 500.000 g/mol eingesetzt werden. Dabei bestimmt sich das geeignete Molekulargewicht einerseits danach, ob eine Paste oder einen Pulverkomponenten hergestellt wird sowie den weiteren Komponenten in der Paste als auch, dadurch dass das Polymer in dem eingesetzten Monomer löslich sein muss.

Die in der Zusammensetzung, u.a. in der Monomerkomponente oder als Monomerkomponente eingesetzten, radikalisch polymerisierbaren Monomere sind vorzugsweise ausgewählt aus mindestens einem Alkyl-2-acrylsäurealkylester, Aryl-2-acrylsäurealkylester, Arylalkyl-2-acrylsäurealkylester jeweils unabhängig mit 1 bis 20 C-Atomen, vorzugsweise 18 C-Atomen in der linearen, verzweigten oder cyclischen Alkyl-Gruppe, insbesondere mit 1 bis 4 C-Atomen, jeweils unabhängig mit 6 bis 13 C-Atomen in der Aryl-Gruppe, jeweils unabhängig mit 6 bis 14 C-Atomen in der Arylalkyl-Gruppe, insbesondere mit 8 bis 12 C-Atomen, und jeweils unabhängig mit 1 bis 20 C-Atomen in der Alkylester-Gruppe, vorzugsweise mit 1 bis 10 C-Atomen in der Alkylester-Gruppen, wobei die Alkylester-Gruppe eine lineare, verzweigte oder cyclische Alkyl-Gruppe aufweisen kann, insbesondere mit 1 bis 4 C-Atomen, oder eine Mischung umfassend mindestens zwei der genannten Monomere ist, wobei Methacrylsäuremethylester, ein Methacrylsäureester oder ein Alkylacrylsäuremethylester bevorzugt sind. Besonders bevorzugt ist Methacrylsäure-methylester, wie ein Methacrylat-Monomer, insbesondere ein Methacrylat-Monomer, welches bei einer Temperatur von 25°C und einem Druck von 1.013 hPa flüssig ist. Vorzugsweise handelt es sich bei dem radikalisch polymerisierbaren Monomer nicht um einen von Bisphenol A abgeleiteten Methacrylsäureester.

Das Methacrylat-Monomer ist vorzugsweise ein Methacrylsäureester. Vorzugsweise handelt es sich bei dem Methacrylsäureester um einen monofunktionellen Methacrylsäureester. Dieser ist vorzugsweise hydrophob. Durch die Verwendung von hydrophoben monofunktionellen Methacrylsäureestern kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält.Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Das erfindungsgemäß eingesetzte radikalisch polymerisierbare Monomer weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer zeichnet sich vorzugsweise dadurch aus, dass eine wässrige Lösung, die vorzugsweise 1 : 1, mit dem radikalisch polymerisierbaren Monomers versetzt wurde einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Gemäß einer besonders bevorzugten Ausführungsform handelt es sich bei dem Methacrylat-Monomer um Methacrylsäuremethylester, Methacrylsäureethylester oder um eine Mischung aus diesen beiden Monomeren.

Vorzugsweise beinhaltet die erfindungsgemäße Paste das radikalisch polymerisierbare Monomer in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der erfindungsgemäßen Paste.

Ebenfalls Gegenstand der Erfindung sind Zusammensetzungen umfassend ein partikuläres, anorganisches Additiv, insbesondere mit einer BET-Oberfläche von mindestens 40 m²/g, wobei vorzugsweise das Additiv kovalent gebundene Hydroxylgruppen aufweist. Erfindungsgemäß geeignete partikuläre anorganische Additive weisen an den Partikeln kovalent gebundene HO-Si-Gruppen (Silanol-Gruppen) auf. Durch diese an der Partikeloberfläche angeordneten Hydroxylgruppen können sich Wasserstoffbrückenbindungen zwischen den Füllstoffpartikeln ausbilden, die durch Einwirkung von mechanischer oder thermischer Energie reversibel gelöst werden können.

Das partikuläre anorganische Additiv ist ausgewählt aus der Gruppe aus pyrogenem Siliziumdioxid, pyrogenem Metall-Siliziummischoxide, Bentonit, Montmorillonit und einer Mischung enthaltend mindestens zwei der genannten Additive.

Daneben ist es auch möglich, hydrophobiertes pyrogenes Siliziumdioxid einzusetzen. Die Herstellung des hydrophoben Siliziumdioxids kann nach dem Stand der Technik durch Behandlung von pyrogenem Siliziumdioxid mit Dialkyldichlorsilanen (z. B. Dimethyldichlorsilan) erfolgen.

Ein ganz besonders bevorzugter partikulärer, anorganischer Füllstoff ist pyrogenes Siliziumdioxid mit einer BET-Oberfläche von mindestens 40 m²/g, weiter bevorzugt von 200 m²/g und am meisten bevorzugt von 300 m²/g. Derartiges pyrogenes Siliziumdioxid ist unter anderem unter dem Markennamen Aerosil® mit spezifischen BET-Oberflächen von 50 m²/g, 90 m²/g, 200 m²/g und 380 m²/g kommerziell verfügbar.

Als partikuläres anorganisches Additiv ist pyrogenes Siliziumdioxid mit einer BET-Oberfläche von mindestens 200 m²/g bevorzugt. Ebenfalls bevorzugt kann als ein partikuläres anorganisches Additiv pyrogenes Siliziumdioxid mit einer BET-Oberfläche von mindestens 300 m²/g eingesetzt werden. Die erfindungsgemäß geeigneten partikulären anorganischen Additive weisen vorzugsweise Primärteilchen von etwa 7 nm und eine spezifische Oberfläche 270 bis 330 m²/g auf.

Die BET-Messung ist ein Analyseverfahren zur Charakterisierung von Oberflächen von Festkörpern mit Hilfe der Gasadsorption. Die Bestimmungsmethode ist in der DIN ISO 9277:2003-05 (Bestimmung der spezifischen Oberfläche von Feststoffen durch Gasadsorption nach dem BET-Verfahren) beschrieben.

Eine erfindungsgemäße Zusammensetzung umfasst neben dem löslichen organischen Polymer, insbesondere Polymethylmethacrylat (PMMA), und dem radikalisch polymerisierbaren Monomer, insbesondere Methacrylsäuremethylester, ein partikuläres, anorganisches Additiv, vorzugsweise in einer Konzentration von 0,01 bis 0,5 Gew.-%, insbesondere von 0,01 bis 0,25 Gew.-%, bevorzugt von 0,02-0,14 Gew.-% in Bezug auf die Gesamtzusammensetzung. Erfindungsgemäß umfasst der aus der Pulverkomponente und der flüssigen Monomerkomponente gemischte Zementteig das partikuläre, anorganische Additiv in einer Konzentration von 0,02-0,14 Gew.-%. Zusätzlich zu den vorgenannten Komponenten umfasst eine erfindungsgemäße Zusammensetzung einen Röntgenopaker, einen Polymerisationsinitiator und/oder einen Polymerisationsbeschleuniger sowie optional zusätzlich Füllstoffe, die nicht dem Additiv entsprechend und lediglich verdickende Wirkung zeigen.

Darüber hinaus kann die Zusammensetzung übliche anorganische oder organische Füllstoffe enthalten, wie Siliciumdioxid mit einer BET-Oberfläche von deutlich kleiner als 35 m²/g.

Entsprechend einer Ausführungsform der Erfindung wird ein Kit, vorzugsweise zur Herstellung einer erfindungsgemäßen Zusammensetzung bzw. Knochenzementes, umfassend eine Paste A und eine Paste B offenbart, wobei
(a) Paste A
   (a1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (a2) mindestens ein in (a1) lösliches organisches Polymer, insbesondere zu 5 bis 50 Gew.-%, bevorzugt 10 bis 40 Gew.-%, besonders bevorzugt 20 bis 30 Gew.-%, und
   (a3) mindestens einen Polymerisationsinitiator; insbesondere zu 0,1 bis 10 Gew.-%, bevorzugt 0,01 bis 8 Gew.-%, besonders bevorzugt 0,01 bis 5 Gew.-%, sowie optional weitere Bestandteile enthält, wie Röntgenopaker und/oder in (a1) unlöslichen Füllstoff, jeweils bezogen auf das Gesamtgewicht der Paste A, und die
(b) Paste B
   (b1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,
   (b2) mindestens ein in (b1) lösliches organisches Polymer, insbesondere zu 5 bis 50 Gew.-%, bevorzugt 10 bis 40 Gew.-%, besonders bevorzugt 20 bis 30 Gew.-%, und
   (b3) mindestens einen Polymerisationsbeschleuniger beinhaltet; insbesondere zu 0,0005 bis 0,5 Gew.-%, sowie optional weitere Bestandteile enthält, wie Röntgenopaker und/oder in (b1) unlöslichen Füllstoff, jeweils bezogen auf das Gesamtgewicht der Paste B, und wobei mindestens eine der Pasten A oder B als Komponente (a4) oder (b4) oder beide Pasten A und B als Komponenten (a4) und (b4) mindestens einen Gehalt einer Wasserstoffperoxid-Verbindung umfasst, vorzugsweise von 0,001 bis 10 Gew.-%, bevorzugt von 0,001 bis 5 Gew.-%, besonders bevorzugt von 0,001 bis 2,5 Gew.-%, weiter bevorzugt zwischen 0,001 bis 1 Gew.-% in Bezug auf die Gesamtzusammensetzung einer Paste.

Dabei kann jede der Pasten das partikuläre, anorganische Additiv in einer Konzentration 0,001 bis 2 Gew.-%, insbesondere 0,001 bis 1 Gew.-% enthalten, so dass in der durch Vermischen der Pasten A und B, insbesondere im Verhältnis von annähernd 1 zu 1 mit jeweils plus/minus 0,5, erhältlichen Zusammensetzung 0,01 bis 0,5 Gew.-% Additiv, insbesondere von 0,01 bis 0,25 Gew.-%, bevorzugt von 0,02-0,14 Gew.-% in Bezug auf die Gesamtzusammensetzung vorliegen.
Entsprechendes gilt für die nachstehende Pulverkomponente C und Paste D.

Als Monomere und Polymere werden die vorstehend definierten in den Pasten A und B eingesetzt.

Entsprechend einer weiteren Ausführungsform wird ein Kit, vorzugsweise zur Herstellung einer erfindungsgemäßen Zusammensetzung bzw., Knochenzementes, umfassend eine Pulverkomponente C und eine Monomerkomponente D offenbart, wobei die
(c) Pulverkomponente C
   (c1) mindestens ein pulverförmiges Polyacrylat, insbesondere zu 1 bis 95 Gew.-%, bevorzugt bis 85 Gew.-%,
   (c2) mindestens einen pulverförmigen Röntgenopaker, insbesondere zu 3 bis 60 Gew.-%, bevorzugt 3 bis 30 Gew.-%, und
   (c3) mindestens einen Polymerisationsinitiator beinhaltet; insbesondere zu 0,1 bis 10 Gew.-%, bevorzugt 0,01 bis 8 Gew.-%, besonders bevorzugt 0,01 bis 5 Gew.-%, sowie optional weitere Bestandteile, wie Röntgenopaker und/oder in (a1) unlöslicher Füllstoff, jeweils bezogen auf das Gesamtgewicht der Pulverkomponente C enthält,
      und die
(d) Monomerkomponente D -
   (d1) wenigstens ein radikalisch polymerisierbares Monomer, insbesondere zu 90 bis 99,9995 Gew.-%,
   (d2) optional wenigstens ein in (d1) lösliches organisches Polymer
   (d3) wenigstens einen Polymerisationsbeschleuniger beinhaltet; insbesondere zu 0,0005 bis 0,5 Gew.-% sowie optional weitere Bestandteile, wie Röntgenopaker und/oder in (d1) unlöslichen Füllstoff, jeweils bezogen auf das Gesamtgewicht der Monomerkomponente D, und wobei mindestens die Pulverkomponente C oder die Monomerkomponente D als Komponente (c4) oder (d4) oder die Pulverkomponente C und die Monomerkomponente D als Komponente (c4) und (d4) mindestens einen Gehalt einer Wasserstoffperoxid-Verbindung umfassen, vorzugsweise von 0,001 bis 10 Gew.-%, bevorzugt von 0,001 bis 5 Gew.-%, besonders bevorzugt von 0,001 bis 2,5 Gew.-%, weiter bevorzugt zwischen 0,001 bis 1 Gew.-%, in Bezug auf eine Gesamtzusammensetzung C oder D.

Dabei werden vorzugsweise als Wasserstoffperoxid-Verbindung die vorgenannten Verbindungen umfassend Wasserstoffperoxid, Wasserstoffperoxid-Addukte, Wasserstoffperoxid-Komplexe, Salze von Wasserstoffperoxid, Co-Kristalle von Wasserstoffperoxid, Wasserstoffperoxid freisetzende Verbindungen oder Gemische enthaltend mindestens zwei der vorgenannten Verbindungen in einer der Pasten A und/oder B oder alternativ in der Pulverkomponenten C und/oder der Monomerkomponente D in den genannten Mengen jeweils bezogen auf 100 Gew.-% der Gesamtzusammensetzung eingesetzt.

Ferner ist Gegenstand der Erfindung ein Kit, in dem in mindestens einer der Pasten A oder B als Komponente (a4) oder (b5) oder in beiden Pasten A und B als Komponenten (a4) und (b5) einen Gehalt einer Puffersubstanz oder ein Gemisch von Puffersubstanzen enthalten ist. Gleichermaßen ist Gegenstand der Erfindung ein Kit, in dem in Pulverkomponente C oder Monomerkomponente D als Komponente (c5) oder (d5) oder sowohl in Pulverkomponente C als auch Monomerkomponente D als Komponenten (c5) und (d5) ein Gehalt einer Puffersubstanz oder ein Gemisch von Puffersubstanzen enthalten ist. Der Gehalt an Puffersubstanz kann zwischen 0,001 bis 10 Gew.-% in der jeweiligen Gesamtzusammensetzung der Pasten oder der Monomer- und Pulverkomponenten betragen, bevorzugt von 0,001 bis 5 Gew.-%, besonders bevorzugt von 0,001 bis 2,5 Gew.-%, weiter bevorzugt zwischen 0,001 bis 1 Gew.-%

Als pulverförmiges Polyacrylat wird ein organisches Polymer in Form eines Pulvers gemäß vorstehender Definition eingesetzt, bevorzugt ist pulverförmiges PMMA. Generell kann das Additiv mit einem Gehalt sowohl in der Pulverkomponente als auch in der Paste vorliegen.

Im Falle einer erfindungsgemäßen Zusammensetzung, welche durch die Kombination zweier Pasten A und B oder der Pulverkomponente C und der Monomerkomponente D eines Zweikomponenten-Systems erhalten wurde, beinhaltet diese Zusammensetzung vorzugsweise mindestens einen Polymerisationsinitiator (der in der einen Paste/Komponente des Zweikomponenten-Systems enthalten war) und wenigstens einen Polymerisationsbeschleuniger (der in der anderen Paste/Komponente des Zweikomponenten-Systems enthalten war).

Als Monomere und Polymere werden die vorstehend definierten in der Pulverkomponente C und der Monomerkomponente D eingesetzt.

Üblicherweise enthalten die Paste A und/oder B sowie die Pulverkomponente C und/oder die Monomerkomponente D jeweils unabhängig voneinander einen Röntgenopaker.

Die vorgenannten Pasten A und B können in jedem beliebigen Verhältnis miteinander gemischt werden, wobei sich die Verwendung der Pasten A und B in einem Verhältnis von im Wesentlichen 1 zu 1, die miteinander gemischt werden können als bevorzugt erwiesen hat, wobei das Verhältnis zueinander unabhängig plus/minus 50 % schwanken kann.

Die erfindungsgemäßen Zusammensetzungen, Pasten und/oder Pulverkomponenten können mindestens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder gegebenenfalls wenigstens einen Co-Polymerisationsinitiator beinhalten.

Im Falle eines Einkomponenten-Systems als erfindungsgemäße Zusammensetzung handelt es sich bei dem Polymerisationsinitiator vorzugsweise um einen aktivierbaren Polymerisationsinitiator, z. B. um einen in der Zusammensetzung, die als Paste vorliegt, gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle. Auch kann im Falle eines Einkomponenten-Systems die erfindungsgemäße Zusammensetzung oder Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Ebenfalls ist Gegenstand der Erfindung ein härtbarer Knochenzement erhältlich durch Vermischen der Pasten A und B oder der Pulverkomponente C mit der Monomerkomponente D sowie auch ein gehärteter Knochenzement, erhältlich durch Polymerisation einer erfindungsgemäßen Zusammensetzung oder erhältlich durch Vermischen und Polymerisation der Pasten A und B oder der Pulverkomponente C mit der Monomerkomponente D, wobei der Knochenzement einem Gehalt an Wasserstoffperoxid-Verbindung von größer gleich 0,01 bis 5 Gew.-%, vorzugsweise von 0,01 bis 1 Gew.-% in Bezug auf die Gesamtzusammensetzung aufweist. Vorzugsweise wird die Wasserstoffperoxid-Verbindung retardiert aus der härtbaren oder der gehärteten Zusammensetzung erst in Gegenwart von Wasser, feuchtem oder wässrigem Milieu freigesetzt, besonders vorzugsweise erst nach der Aushärtung.

Ferner ist Gegenstand der Erfindung ein gehärteter Knochenzement, wobei a) die Wasserstoffperoxid-Verbindung, wie vorstehend definiert, vorzugsweise ein Wasserstoffperoxid Addukt, ein Wasserstoffperoxid Komplex, ein Wasserstoffperoxid Co-Kristall oder ein Wasserstoffperoxid Salz ist, dass in Gegenwart von Feuchte, Wasser, in wässrigem Milieu oder einer wässrigen Lösung Wasserstoffperoxid als oxidierend wirkende Verbindung freisetzt und/oder b) eine Wasserstoffperoxid-Verbindung ist, durch Enzyme katalysiert Wasserstoffperoxid freisetzt, wobei in a) und b) das Wasserstoffperoxid retardiert freigesetzt wird

Auch ein Formkörper erhältlich durch Polymerisation einer erfindungsgemäßen Zusammensetzung oder erhältlich durch Vermischen der Pasten A und B oder der Pulverkomponente C mit der Monomerkomponente D und Durchführen einer Polymerisation ist Gegenstand der Erfindung. Dabei ist es weiter bevorzugt, wenn im ausgehärteten Formkörper die Wasserstoffperoxid-Verbindung erst durch den Kontakt mit Wasser, Feucht oder einem wässrigen Milieu, wie der Körperflüssigkeit, Wasserstoffperoxid freisetzt. Besonders bevorzugt umfasst der ausgehärtete Formkörper die Wasserstoffperoxid-Verbindung zu 0,01 bis 5 Gew.-% in Bezug auf den gesamten ausgehärteten Formkörper, wie vorzugsweise das wenigstens eine Addukt oder Salz des Wasserstoffperoxids im ausgehärteten Polymethylmethacrylat-Knochenzement.

Gleichfalls ist Gegenstand der Erfindung die Verwendung einer erfindungsgemäßen Zusammensetzung oder eines erfindungsgemäßen Kits, zur Herstellung eines Implantats, eines antiseptischen Implantats, eines Revisionsimplantats, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für lokale Freisetzung von pharmazeutisch wirksamen Substanzen.

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind. Die Polymerisationsinitiatoren zerfallen radikalisch, ggf. in der Regel unter Bildung von Wasserstoffradikalen und Abspaltung von Sauerstoff. In Gegenwart von Wasser bildet der Initiator kein Wasserstoffperoxid. Cumol-hydroperoxid kann in Gegenwart von Wasser zu Keton und Phenol umlagern. Radikale wie H-O-O•, R-O-O•, R = organischer Rest, gelten nicht als Wasserstoffperoxid

Unter einem Peroxid werden erfindungsgemäß Verbindungen verstanden, die wenigstens eine Peroxogruppe (-O-O-) enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Das Hydroperoxid ist jedoch kein Wasserstoffperoxid. Gemäß einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amyl-hydroperoxid, Di-Isopropylbenzenmono-hydroperoxid und einer Mischung aus mindestens zwei davon besteht.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten-und 1,3,5-trisubstituierten Barbituraten. Gemäß einer besonderen Ausgestaltung der erfindungsgemäßen Paste ist das Barbitursäurederivat ausgewählt aus der Gruppe bestehend aus 1,5-disubstituierten Barbituraten-und 1,3,5-trisubstituierten Barbituraten.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf. Erfindungsgemäß bevorzugt sind Barbiturate, die an Position 1 und an Position 5 jeweils einen Substituenten oder an den Positionen 1, 3 und 5 jeweils einen Substituenten -tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt. Erfindungsgemäß bevorzugte Schwermetallverbindungen sind ausgewählt aus der Gruppe bestehend Kupfer(II)-hydroxid, Kupfer(II)methacrylat, Kupfer(II)acetylacetonat, Kupfer(II)-2-ethylhexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform der erfindungsgemäßen Zusammensetzung oder Paste ist der Polymerisationsbeschleuniger ausgewählt aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäurediimid und einer Mischung aus mindestens zwei davon.

Eine weitere vorteilhafte Ausgestaltung der Erfindung umfasst eine Verwendung von Kombinationen aus Schwermetallsalzen und mindestens einem Vertreter aus der Gruppe umfassend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei werden Zweifachkombinationen und auch Dreifachkombinationen von unterschiedlichen Polymerisationsbeschleunigern im Rahmen der Erfindung offenbart.

Eine vorteilhafte Ausgestaltung der Erfindung besteht darin, dass gegebenenfalls wenigstens ein Co-Polymerisationsbeschleuniger in der erfindungsgemäßen Zusammensetzung oder in einer der Pasten A, B oder der Monomerkomponente D enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethyl-anilin, 1,8-Diazabicyclo[5.4.0-]undec-7-en und 1,5-Diazabicyclo(4.3.0)-non-5-en als Co-Akzeleratoren bevorzugt sind.

Die erfindungsgemäße Zusammensetzung, insbesondere in einer Paste oder Monomerkomponente kann den Polymerisationsinitiator, den Polymerisationsbeschleuniger, den Co-Polymerisationsbeschleuniger oder den Polymerisationsinitiator, den Polymerisationsbeschleuniger und den Co-Polymerisationsbeschleuniger in einer (Gesamt)Menge von bis zu 10 Gew.-%, bezogen auf das Gesamtgewicht der erfindungsgemäßen Zusammensetzung oder jeweils unabhängig bezogen auf das Gesamtgewicht einer der Pasten A, B oder der Monomerkomponente D beinhalten.

Die erfindungsgemäße Zusammensetzung und insbesondere die Pasten A und B als auch Monomerkomponente D und Pulverkomponente C können neben den vorstehend genannten Komponenten weitere Bestandteile beinhalten.

Gemäß einer bevorzugten Ausführungsform der erfindungsgemäßen Zusammensetzung oder einer der Pasten A oder B sowie der Monomerkomponente D oder der Pulverkomponente C können diese jeweils unabhängig voneinander mindestens einen Röntgenopaker beinhalten. Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentrationen an Röntgenopaker, insbesondere die Konzentration an Zirkoniumdioxid, in der erfindungsgemäßen Zusammensetzung oder einer der Pasten A oder B sowie in der Pulverkomponente C oder der Monomerkomponente D können jeweils voneinander unabhängig beispielsweise in einem Bereich von 3 bis 30 Gew.-% in Bezug auf die jeweilige Gesamtzusammensetzung liegen. Röntgenopaker gelten vorliegend nicht als Füllstoffe.

Gemäß einer weiteren bevorzugten Ausführungsform kann die erfindungsgemäße Zusammensetzung oder eine der vorgenannten Pasten mindestens einen Farbstoff beinhalten. Der Farbstoff kann ein fachüblicher Farbstoff und vorzugsweise ein Lebensmittelfarbstoff sein. Ferner kann der Farbstoff in dem wenigstens einem radikalisch polymerisierbaren Monomer löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132. Gemäß einer weiteren bevorzugten Ausführungsform kann die erfindungsgemäße Zusammensetzung mindestens ein biokompatibles Elastomer beinhalten. Das biokompatible Elastomer ist vorzugsweise partikulär. Vorzugsweise ist das biokompatible Elastomer in dem wenigstens einen radikalisch polymerisierbaren Monomer löslich. Als besonders geeignet hat sich die Verwendung von Polybutadien als biokompatibles Elastomer erwiesen.

Gemäß einer weiteren bevorzugten Ausführungsform kann die erfindungsgemäße Zusammensetzung mindestens ein Monomer mit Haftgruppen beinhalten. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform kann die erfindungsgemäße Zusammensetzung oder mindestens eine der Pasten A oder B oder die Monomerkomponente D mindestens einen Stabilisator beinhalten. Der Stabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemäßen Paste enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Erfindungsgemäß wird unter Kit ein System aus wenigstens zwei Komponenten verstanden. Obwohl im Folgenden von zwei Komponenten (z. B. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Vorzugsweise ist der Kit als Vorrichtung zur Herstellung von Zusammensetzungen zur Verwendung als Knochenzement so ausgestaltet, dass es einen ersten Behälter und einen zweiten Behälter aufweist, wobei der erste Behälter zum Beispiel die Paste A und der zweite Behälter die Paste B aufweist, wobei wenigstens einer der Behälter zu öffnen ist, um nach dem Öffnen ein Vermischen der Pasten A und B zu gestatten, und eine Mischeinheit zum Vermischen der Pasten A und B. Entsprechend kann das Kit als Vorrichtung zur Herstellung der erfindungsgemäßen Zusammensetzung einen ersten Behälter für die Pulverkomponente C und einen zweiten Behälter für die Monomerkomponente D aufweisen.

Im Falle des ersten Kits werden dazu die wenigstens zwei Pasten A und B miteinander vermischt, wobei die erfindungsgemäße Zusammensetzung erhalten wird. Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B. Das Mischungsverhältnis der Pulverkomponente C und der Monomerkomponente D beträgt vorzugsweise 3 zu 1 bis 1 zu 2, insbesondere um 2 zu 1 Gewichtsteile. Das Mischen kann mit üblichen Mischgeräten, beispielsweise einem statischen Mischer oder einem dynamischen Mischer erfolgen.

Nach dem Vermischen der Pasten des Kits ist die letztlich erhaltene Zusammensetzung spätestens nach 1 bis 2 Minuten nach der Norm ISO 5833 klebfrei. Der erfindungsgemäße antiseptische Polymethylmethacrylat-Knochenzement wird als Zement zur mechanischen Fixierung von primären Totalgelenkendoprothesen, zur mechanischen Fixierung von Revisions-Totalgelenkendoprothesen und zur Herstellung von Spacern verwendet. Die Erfindung wird durch nachstehende Beispiele näher erläutert, ohne jedoch die Erfindung zu beschränken.

Es wurden die folgenden Beispiele unter Verwendung von Palacos® R-Zementpulvers (Charge B7457), ein PMMA-Zementpulver, durchgeführt. Harnstoff-Peroxid-Addukt und das Calciumperoxid wurden von Sigma-Aldrich bezogen.

Es wurden jeweils 10,0 g Palacos® R-Zementpulver mit fein gepulvertem Harnstoffperoxid-Addukt bzw. Calciumperoxid in 100 ml Plastik-Gefäße mit Schraubdeckel eingewogen und anschließend 30 Minuten in einem Turbula-Mischer gemischt. Die Zusammensetzung der Beispiele 1-9 ist in den beiden nachfolgenden Tabellen angegeben. Die Beispiele 6-8 sind erfingungsgemäss, Beispiele 1-5 dienen zum Vergleich.

Anschließend wurden die Gemische mit jeweils 5 ml Palacos-Monomerflüssigkeit, eine MMA-Flüssigkeit, vermischt. Es bildete sich innerhalb von 60 Sekunden ein klebfreier Zementteig. Dieser wurden anschließend in zylindrische Kunststoffformen gestrichen. Nach erfolgter Aushärtung wurden die zylindrischen Probekörper (Höhe 12 mm, Durchmesser 6 mm) entnommen.

| Beispiele | Zementpulver-Zusammensetzung | |
|---|---|---|
| | Palacos® R-Zementpulver [g] | Harnstoff-Peroxid-Addukt [g] |
| 1 | 10,0 | 1,50 |
| 2 | 10,0 | 1,00 |
| 3 | 10,0 | 0,75 |
| 4 | 10,0 | 0,50 |
| 5 | 10,0 | 0,25 |

| Beispiele | Zementpulver-Zusammensetzung | |
|---|---|---|
| | Palacos® R-Zementpulver [g] | Calciumperoxid [g] |
| 6 | 10,0 | 1,00 |
| 7 | 10,0 | 0,50 |
| 8 | 10,0 | 0,25 |

Als Referenz (Beispiel 10) wurde reines Palacos® R-Pulver mit 5 ml Monomerflüssigkeit vermischt und es wurden ebenfalls zylindrische Probekörper (Höhe 12 mm, Durchmesser 6 mm) hergestellt.

Die Probekörper wurden von der QualityLabs BT GmbH Nürnberg auf ihre antimikrobielle Aktivität hinuntersucht (Report Measurement 20120807-RB01-10). Dabei kam der MRSA-Teststamm Staphylococcus aureus DSM 21979/EDCC 5247 als Testkeim zur Anwendung. Die antimikrobielle Wirksamkeit wurde gemäß der SOP für Staphylococcus Epidermis gemäß SOP 3.2 aus 2008-08-05 an dem MRSA-Teststamm Staphylococcus aureus durchgeführt. Es wurde der Certika-Proliferations-Test durchgeführt. Dazu wurden die Probekörper zuerst in Bakteriensuspension inkubiert. Danach wurden die Probekörper gewaschen und die Probekörper wurden 18 Stunden mit frischem Nährmedium bei 37 °C inkubiert. Danach wurde ein 200 µl-Aliquot entnommen und in eine Mikrotiter-Platte überführt. Die optische Dichte wurde anschließend im Abstand von jeweils 30 Minuten über einen Zeitraum von 48 Stunden gemessen. Bei einem mikrobiellen Wachstum steigt die optische Dichte zeitabhängig an. Ziel war es die Proliferation des Bakterium auf der Testoberfläche zu detektieren.

Die Aliquote der Probekörper der Beispiele 1-9 zeigten innerhalb von 48 Stunden keinerlei Wachstum des Testkeims, wie es in der nachfolgenden Tabelle dargestellt ist. Die Bakterien konnten im umgebenden Medium bei 37 °C innerhalb einer Zeitspanne von größer gleich 48 Stunden nicht proliferieren und konnten keine Tochterzellen bilden. Das bedeutet, dass die Probekörper eine ausgeprägte antimikrobielle Wirkung haben.

Die Eluate der Referenzprobekörper des Beispiels 10 behinderten dagegen das Wachstum des Testkeims nicht.

| Beispiel | Onset OD [h] | Onset OD [h] | Standardabweichung | CV [%] | Ergebnis |
|---|---|---|---|---|---|
| Referenzprobe körper | 12.6 | - | 1.2 | 9.3 | |
| 1 | > 48.0 | > 48.0 | 0.0 | 0.0 | antimikrobiell |
| 2 | > 48.0 | > 48.0 | 0.0 | 0.0 | antimikrobiell |
| 3 | > 48.0 | > 48.0 | 0.0 | 0.0 | antimikrobiell |
| 4 | > 48.0 | > 48.0 | 0.0 | 0.0 | antimikrobiell |
| 5 | > 48.0 | > 48.0 | 0.0 | 0.0 | antimikrobiell |
| 6 | > 48.0 | > 48.0 | 0.0 | 0.0 | antimikrobiell |
| 7 | > 48.0 | > 48.0 | 0.0 | 0.0 | antimikrobiell |
| 8 | > 48.0 | > 48.0 | 0.0 | 0.0 | antimikrobiell |

## Patentansprüche

1. Zusammensetzung zur Verwendung als antiseptisch wirkendem Polymethylmethacrylat-Knochenzement, wobei die Zusammensetzung härtbar ist und einen Gehalt mindestens einer Komponente aufweist, aus der eine oxidierend wirkende Verbindung freisetzbar ist, wobei die oxidierende Verbindung in Gegenwart von Wasser aus der Komponente freisetzbar ist, wobei die Komponente eine Wasserstoffperoxid-Verbindung umfasst, wobei die Wasserstoffperoxid-Verbindung ein Calciumperoxid oder ein Calciumperoxid-Octahydrat ist, wobei die Verwendung **dadurch gekennzeichnet ist, dass** Wasserstoffperoxid nach einer Implantation durch Einwirkung von Körperflüssigkeiten retardiert aus der gehärteten Zusammensetzung durch Hydrolyse freigesetzt wird.

2. Zusammensetzung zur Verwendung gemäß Anspruch 1, **dadurch gekennzeichnet, dass** die Wasserstoffperoxid-Verbindung ein Gemisch umfassend ein Harnstoff-Wasserstoffperoxid-Addukt und Calciumperoxid ist.

3. Zusammensetzung zur Verwendung gemäß Anspruch 1 oder 2, **dadurch gekennzeichnet, dass** die Wasserstoffperoxid-Verbindung zu 0,01 bis 10 Gew.-% in der Gesamtzusammensetzung enthalten ist.

4. Zusammensetzung zur Verwendung gemäß einem der Ansprüche 1 bis 3, **dadurch gekennzeichnet, dass** die Zusammensetzung mindestens eine Puffersubstanz umfasst.

5. Zusammensetzung zur Verwendung gemäß Anspruch 4, **dadurch gekennzeichnet, dass** die Zusammensetzung mindestens eine Puffersubstanz oder ein Gemisch von Puffersubstanzen umfasst, die ausgewählt sind aus der Gruppe der primären Alkaliphosphate, der sekundären Alkaliphosphate, der primären Alkalicitrate, der sekundären Alkalicitrate und/oder aus der Gruppe der Alkali-, Erdalkali- und/oder Zink-Salze von Carbonsäuren mit 1 bis 20 C-Atomen, Fruchtsäuren, alpha-Hydroxysäuren, Fettsäuren, Citronensäure, Aepfelsäure, Weinsäure und Milchsäure.

6. Zusammensetzung zur Verwendung gemäß Anspruch 4 oder 5, **dadurch gekennzeichnet, dass** die Wasserstoffperoxid-Verbindung vorliegt als i) ein Addukt mit einer Puffersubstanz, ii) in einem Co-Kristall zusammen mit der Puffersubstanz, iii) in einer Formulierung zusammen mit mindestens einer Puffersubstanz oder iv) im Gemisch mit mindestens einer Puffersubstanz.

7. Zusammensetzung zur Verwendung gemäß einem der Ansprüche 1 bis 6, **dadurch gekennzeichnet, dass** die Zusammensetzung ein antiseptischer Polymethylmethacrylat-Knochenzement ist.

8. Zusammensetzung zur Verwendung gemäß einem der Ansprüche 1 bis 7, **dadurch gekennzeichnet, dass** die Zusammensetzung mindestens ein radikalisch polymerisierbares Monomer und mindestens ein organisches Polymer umfasst, wobei das Polymer in dem Monomer löslich ist.

9. Zusammensetzung zur Verwendung gemäß einem der Ansprüche 1 bis 8, **dadurch gekennzeichnet, dass** die Zusammensetzung mindestens ein partikuläres, anorganisches Additiv umfasst, insbesondere mit einer BET-Oberfläche von mindestens 40 m2/g, wobei vorzugsweise das Additiv kovalent gebundene Hydroxylgruppen aufweist.

10. Zusammensetzung zur Verwendung gemäß einem der Ansprüche 1 bis 8, **dadurch gekennzeichnet, dass** das organische Polymer ausgewählt ist aus Poly(alkyl-2-acrylsäure-alkylester), Poly(aryl-2-acrylsäurealkylester), Poly(arylalkyl-2-acrylsäure-alkylester) jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Polymere.

11. Zusammensetzung zur Verwendung gemäß einem der Ansprüche 8 bis 10, **dadurch gekennzeichnet, dass** das organische Polymer ausgewählt ist aus der Gruppe Poly(methacrylsäuremethylester), Poly(methacrylsäureethylester), Poly(methylmethacrylsäurepropylester), Poly(methacrylsäureisopropylester), Poly(methylmethacrylat-co-methylacrylat), Poly(styrol-co-methyl-meth-acrylat), Copolymeren der genannten Verbindungen und einer Mischung umfassend mindestens zwei dieser Polymere.

12. Zusammensetzung zur Verwendung gemäß einem der Ansprüche 7 bis 9, **dadurch gekennzeichnet, dass** das Monomer ausgewählt ist aus mindestens einem Alkyl-2-acrylsäurealkylester, Aryl-2-acrylsäurealkylester, Arylalkyl-2-acrylsäurealkylester jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere.

13. Zusammensetzung zur Verwendung gemäß einem der Ansprüche 7 bis 11, **dadurch gekennzeichnet, dass** das organische Polymer mindestens ein Poly(methacryl-säuremethylester), (PMMA) und als Monomer Methacrylsäuremethylester (MMA) umfasst.

14. Kit aufweisend eine Paste A und eine Paste B, wobei (a) Paste A (a1) mindestens ein radikalisch polymerisierbares Monomer, (a2) mindestens ein in (a1) lösliches organisches Polymer, und (a3) mindestens einen Polymerisationsinitiator beinhaltet; (b) Paste B (b1) mindestens ein radikalisch polymerisierbares Monomer, (b2) mindestens ein in (b1) lösliches organisches Polymer, und (b3) mindestens einen Polymerisationsbeschleuniger beinhaltet;und wobei mindestens eine der Pasten A und/oder B als Komponente (a4) bzw. (b4) mindestens einen Gehalt einer Wasserstoffperoxid-Verbindung umfasst, wobei die Wasserstoffperoxid-Verbindung ein Calciumperoxid oder ein Calciumperoxid-Octahydrat ist, zur Verwendung als antiseptisch wirkender Polymethylmethacrylat-Knochenzement, wobei die Verwendung **dadurch gekennzeichnet ist, dass** Wasserstoffperoxid nach einer Implantation durch Einwirkung von Körperflüssigkeiten retardiert aus der gehärteten Zusammensetzung durch Hydrolyse freigesetzt wird.

15. Kit aufweisend eine Pulverkomponente C und eine - Monomerkomponente D, wobei die(c) Pulverkomponente C(c1) mindestens ein pulverförmiges Polyacrylat, (c2) mindestens einen pulverförmigen Röntgenopaker, und(c3) mindestens einen Polymerisationsinitiator beinhaltet;(d) -Monomerkomponente D (d1) wenigstens ein radikalisch polymerisierbares Monomer, (d2) optional wenigstens ein in (d1) lösliches organisches Polymer- und (d3) wenigstens einen Polymerisationsbeschleuniger beinhaltet;und wobei mindestens die Pulverkomponente C und/oder die Monomerkomponente D als Komponente (c4) bzw. (d4) mindestens einen Gehalt einer Wasserstoffperoxid-Verbindung umfasst, wobei die Wasserstoffperoxid-Verbindung ein Calciumperoxid oder ein Calciumperoxid-Octahydrat ist, zur Verwendung als antiseptisch wirkender Polymethylmethacrylat-Knochenzement, wobei die Verwendung **dadurch gekennzeichnet ist, dass** Wasserstoffperoxid nach einer Implantation durch Einwirkung von Körperflüssigkeiten retardiert aus der gehärteten Zusammensetzung durch Hydrolyse freigesetzt wird.

16. Kit zur Verwendung gemäß einem der Ansprüche 14 oder 15, **dadurch gekennzeichnet, dass** mindestens eine der Pasten A und/oder B als Komponente (a4) bzw. (b5) oder mindestens die Pulverkomponente C und/oder die Monomerkomponente D als Komponente (c5) bzw. (d5) mindestens einen Gehalt einer Puffersubstanz oder ein Gemisch von Puffersubstanzen umfasst.

## Claims

1. A composition for use as polymethylmethacrylate bone cement having an antiseptic effect, wherein the composition is curable and has a content of at least one component, from which a compound having an oxidizing effect can be released, wherein the oxidizing compound can be released from the component in the presence of water, wherein the component comprises a hydrogen peroxide compound, wherein the hydrogen peroxide compound is a calcium peroxide or a calcium peroxide octahydrate, wherein the use is **characterised in that** hydrogen peroxide is released with delay from the cured composition by hydrolysis after an implantation by the effect of body fluids

2. The composition for use according to claim 1, **characterised in that** the hydrogen peroxide compound is a mixture comprising a urea-hydrogen peroxide adduct and calcium peroxide.

3. The composition for use according to claim 1 or 2, **characterised in that** the total composition contains 0.01 to 10% by weight of the hydrogen peroxide compound.

4. The composition for use according to any one of claims 1 to 3, **characterised in that** the composition comprises at least one buffer substance.

5. The composition for use according to claim 4, **characterised in that** the composition comprises at least one buffer substance or a mixture of buffer substances, which are selected from the group of the primary alkali phosphates, the secondary alkali phosphates, the primary alkali citrates, the secondary alkali citrates, and/or from the group of the alkali, alkaline earth and/or zinc salts of carboxylic acids comprising 1 to 20 C atoms, fruit acids, alpha-hydroxy acids, fatty acids, citric acid, malic acid, tartaric acid, and lactic acid.

6. The composition for use according to claim 4 or 5, **characterised in that** the hydrogen peroxide compound is present as i) an adduct comprising a buffer substance, ii) in a co-crystal together with the buffer substance, iii) in a formulation together with at least one buffer substance, or iv) in admixture with at least one buffer substance.

7. The composition for use according to any one of claims 1 to 6, **characterised in that** the composition is an antiseptic polymethylmethacrylate bone cement.

8. The composition for use according to any one of claims 1 to 7, **characterised in that** the composition comprises at least one radically polymerizable monomer and at least one organic polymer, wherein the polymer is soluble in the monomer.

9. The composition for use according to any one of claims 1 to 8, **characterised in that** the composition comprises at least one particulate inorganic additive, in particular comprising a BET surface of at least 40 m2/g, wherein the additive preferably has covalently bound hydroxyl groups.

10. The composition for use according to any one of claims 1 to 8, **characterised in that** the organic polymer is selected from poly(alkyl-1-acrylic acid alkyl ester, poly(aryl-2-acrylic acid alkyl ester), poly(arylalkyl-2-acrylic acid alkyl ester), each independently comprising 1 to 20 C atoms in the alkyl group, each independently comprising 6 to 14 C-atoms in the aryl group, each independently comprising 6 to 14 C atoms in the arylalkyl group, and each independently comprising 1 to 10 C atoms in the alkyl ester group or a mixture comprising at least two of the mentioned polymers.

11. The composition for use according to any one of claims 8 to 10, **characterised in that** the organic polymer is selected from the group of poly(methacrylic acid methylester), poly(methacrylic acid ethylester), poly(methylmethacrylic acid propylester, poly(methacrylic acid isopropylester), poly(methyl methacrylate-co-methyl acrylate), poly(styrene-co-methylmethacrylate), copolymers of the mentioned compounds and a mixture comprising at least two of these polymers.

12. The composition for use according to any one of claims 7 to 9, **characterised in that** the monomer is selected from at least one alkyl-2-acrylic acid alkyl ester, aryl-2-acrylic acid alkyl ester, arylalkyl-2-acrylic acid alkyl ester, each independently comprising 1 to 20 C atoms in the alkyl group, each independently comprising 6 to 14 C atoms in the aryl group, each independently comprising 6 to 14 C atoms in the arylalkyl group, and each independently comprising 1 to 10 C atoms in the alkyl ester group, or a mixture comprising at least two of the mentioned monomers.

13. The composition for use according to any one of claims 7 to 11, **characterised in that** the organic polymer comprises at least one poly(methacrylic acid methylester), (PMMA), and methacrylic acid methylester (MMA) as monomer.

14. A kit having a paste A and a paste B, wherein (a) paste A includes (a1) at least one radically polymerizable monomer, (a2) at least one organic polymer, which is soluble in (a1), and (a3) at least one polymerisation initiator; (b) paste B includes (b1) at least one radically polymerizable monomer, (b2) at least one organic polymer, which is soluble in (b1), and (b3) at least one polymerisation accelerator; and wherein at least one of the pastes A and/or B comprises as component (a4) or (b4), respectively, at least a content of a hydrogen peroxide compound, wherein the hydrogen peroxide compound is a calcium peroxide or a calcium peroxide octahydrate, for use as polymethylmethacrylate bone cement having an antiseptic effect, wherein the use is **characterised in that** hydrogen peroxide is released with delay from the cured composition by hydrolysis after an implantation by the effect of body fluids.

15. A kit having a powder component C and a monomer component D, wherein the (c) powder component C(c1) includes at least one polyacrylate in powder form, (c2) at least one radiopaquer in powder form, and (c3) at least one polymerisation initiator; (d) monomer component D includes (d1) at least one radically polymerizable monomer, (d2) optionally at least one organic polymer, which is soluble in (d1), and (d3) at least one polymerisation accelerator; and wherein at least the powder component C and/or the monomer component D comprises as component (c4) or (d4), respectively), at least a content of a hydrogen peroxide compound, wherein the hydrogen peroxide compound is a calcium peroxide or a calcium peroxide octahydrate, for use as polymethylmethacrylate bone cement having an antiseptic effect, wherein the use is **characterised in that** hydrogen peroxide is released with delay from the cured composition by hydrolysis after an implantation by the effect of body fluids.

16. The kit for use according to any one of claims 14 or 15, **characterised in that** at least one of the pastes A and/or B comprises as component (a4) or (b5), respectively, or at least the powder component C and/or the monomer component D comprises as component (c5) or (d5), respectively, at least a content of a buffer substance or a mixture of buffer substances.

## Revendications

1. Composition pour l'utilisation en tant que ciment osseux en polyméthacrylate de méthyle à action antiseptique, dans laquelle la composition est durcissable et présente une teneur en au moins un composant duquel un composé à action oxydante peut être libéré, dans laquelle le composé oxydant peut être libéré du composant en présence d'eau, dans laquelle le composant comprend un composé de peroxyde d'hydrogène, dans laquelle le composé de peroxyde d'hydrogène est un peroxyde de calcium ou un octahydrate de peroxyde de calcium, dans laquelle l'utilisation est **caractérisée en ce que** du peroxyde d'hydrogène est libéré de la composition durcie par hydrolyse de manière retardée après une implantation par action de fluides corporels.

2. Composition pour l'utilisation selon la revendication 1, **caractérisée en ce que** le composé de peroxyde d'hydrogène est un mélange comprenant un produit d'addition d'urée-peroxyde d'hydrogène et du peroxyde de calcium.

3. Composition pour l'utilisation selon la revendication 1 ou 2, **caractérisée en ce que** le composé de peroxyde d'hydrogène est contenu à 0,01 à 10 % en poids dans la composition globale.

4. Composition pour l'utilisation selon une des revendications 1 à 3, **caractérisée en ce que** la composition comprend au moins une substance tampon.

5. Composition pour l'utilisation selon la revendication 4, **caractérisée en ce que** la composition comprend au moins une substance tampon ou un mélange de substances tampons qui sont sélectionnées parmi le groupe des phosphates alcalins primaires, des phosphates alcalins secondaires, des citrates alcalins primaires, des citrates alcalins secondaires et/ou parmi le groupe des sels alcalins, alcalino-terreux et/ou de zinc d'acides carboxyliques avec 1 à 20 atomes de C, acides de fruits, acides alpha-hydroxyliques, acides gras, acide citrique, acide malique, acide tartrique et acide lactique.

6. Composition pour l'utilisation selon la revendication 4 ou 5, **caractérisée en ce que** le composé de peroxyde d'hydrogène est présent en tant que i) un produit d'addition avec une substance tampon, ii) dans un co-cristal avec la substance tampon, iii) dans une formulation avec au moins une substance tampon ou iv) dans le mélange avec au moins une substance tampon.

7. Composition pour l'utilisation selon une des revendications 1 à 6, **caractérisée en ce que** la composition est un ciment osseux en polyméthacrylate de méthyle antiseptique.

8. Composition pour l'utilisation selon une des revendications 1 à 7, **caractérisée en ce que** la composition comprend au moins un monomère polymérisable par voie radicalaire et au moins un polymère organique, dans laquelle le polymère est soluble dans le monomère.

9. Composition pour l'utilisation selon une des revendications 1 à 8, **caractérisée en ce que** la composition comprend au moins un additif inorganique particulaire, notamment avec une surface spécifique d'au moins 40 m2/g, dans laquelle l'additif présente de préférence des groupes hydroxyle liés par covalence.

10. Composition pour l'utilisation selon une des revendications 1 à 8, **caractérisée en ce que** le polymère organique est sélectionné parmi l'ester alkylique d'acide poly(alkyl-2-acrylique), l'ester alkylique d'acide poly(aryl-2-acrylique), l'ester alkylique d'acide poly(arylalkyl-2-acrylique) à chaque fois indépendamment avec 1 à 20 atomes de C dans le groupe alkyle, à chaque fois indépendamment avec 6 à 14 atomes de C dans le groupe aryle, à chaque fois indépendamment avec 6 à 14 atomes de C dans le groupe arylalkyle et à chaque fois indépendamment avec 1 à 10 atomes de C dans le groupe ester alkylique ou un mélange comprenant au moins deux des polymères cités.

11. Composition pour l'utilisation selon une des revendications 8 à 10, **caractérisée en ce que** le polymère organique est sélectionné parmi le groupe ester méthylique d'acide poly(méthacrylique), ester éthylique d'acide poly(méthacrylique), ester propylique d'acide poly(méthacrylique), ester isopropylique d'acide poly(méthacrylique), poly(méthacrylate) de méthyle-co-acrylate de méthyle, poly(styrène-co-méthacrylate de méthyle), copolymères des composés cités et un mélange comprenant au moins deux de ces polymères.

12. Composition pour l'utilisation selon une des revendications 7 à 9, **caractérisée en ce que** le monomère est sélectionné parmi au moins un ester alkylique d'acide alkyl-2-acrylique, ester alkylique d'acide aryl-2-acrylique, ester alkylique d'acide arylalkyl-2-acrylique à chaque fois indépendamment avec 1 à 20 atomes de C dans le groupe alkyle, à chaque fois indépendamment avec 6 à 14 atomes de C dans le groupe aryle, à chaque fois indépendamment avec 6 à 14 atomes de C dans le groupe arylalkyle et à chaque fois indépendamment avec 1 à 10 atomes de C dans le groupe ester alkylique ou un mélange comprenant au moins deux des monomères cités.

13. Composition pour l'utilisation selon une des revendications 7 à 11, **caractérisée en ce que** le polymère organique comprend au moins un ester méthylique d'acide poly(méthacrylique) (PMMA) et en tant que monomère l'ester méthylique d'acide méthacrylique (MMA).

14. Kit présentant une pâte A et une pâte B, dans lequel (a) la pâte A contient (a1) au moins un monomère polymérisable par voie radicalaire, (a2) au moins un polymère organique soluble dans (a1) et (a3) au moins un initiateur de polymérisation ; (b) la pâte B contient (b1) au moins un monomère polymérisable par voie radicalaire, (b2) au moins un polymère organique soluble dans (b1) et (b3) au moins un accélérateur de polymérisation ; et dans lequel au moins une des pâtes A et/ou B comprend en tant que composant (a4) ou (b4) au moins une teneur en un composé de peroxyde d'hydrogène, dans lequel le composé de peroxyde d'hydrogène est un peroxyde de calcium ou un octahydrate de peroxyde de calcium, pour l'utilisation en tant que ciment osseux en polyméthacrylate de méthyle à action antiseptique, dans lequel l'utilisation est **caractérisée en ce que** du peroxyde d'hydrogène est libéré de la composition durcie par hydrolyse de manière retardée après une implantation par action de fluides corporels.

15. Kit présentant un composant pulvérulent C et un composant de monomère D, dans lequel (c) le composant pulvérulent C contient (c1) au moins un polyacrylate pulvérulent, (c2) au moins un opacifiant radiographique pulvérulent et (c3) au moins un initiateur de polymérisation ; (d) le composant de monomère D contient (d1) au moins un monomère polymérisable par voie radicalaire, (d2) en option au moins un polymère organique soluble dans (d1) et (d3) au moins un accélérateur de polymérisation ; et dans lequel au moins le composant pulvérulent C et/ou le composant de monomère D comprend en tant que composant (c4) ou (d4) au moins une teneur en un composé de peroxyde d'hydrogène, dans lequel le composé de peroxyde d'hydrogène est un peroxyde de calcium ou un octahydrate de peroxyde de calcium, pour l'utilisation en tant que ciment osseux en polyméthacrylate de méthyle à action antiseptique, dans lequel l'utilisation est **caractérisée en ce que** du peroxyde d'hydrogène est libéré de la composition durcie par hydrolyse de manière retardée après une implantation par action de fluides corporels.

16. Kit pour l'utilisation selon une des revendications 14 ou 15, **caractérisé en ce qu'**au moins une des pâtes A et/ou B comprend en tant que composant (a4) ou (b5) ou au moins le composant pulvérulent C et/ou le composant de monomère D en tant que composant (c5) ou (d5) au moins une teneur en une substance tampon ou un mélange de substances tampons.
